# EUROPÄISCHE PATENTSCHRIFT

(11) **EP 1 362 094 B1**
(45) Veröffentlichungstag und Bekanntmachung des Hinweises auf die Patenterteilung: **10.09.2008**
(21) Anmeldenummer: 02714008.6
(22) Anmeldetag: 14.02.2002
(51) Int. Cl.: C12N 5/02, C12N 5/08, C12N 5/06

(54) **MEDIUM ZUR ZÜCHTUNG VON TUMORZELLEN**
MEDIUM FOR CULTURING TUMOUR CELLS
MILIEU DE CULTURE DE CELLULES TUMORALES

(30) Priorität: 14.02.2001 DE 10106826
(43) Veröffentlichungstag der Anmeldung: 19.11.2003
(73) Patentinhaber: Uni-Klinikum Hamburg-Eppendorf, 20246 Hamburg (DE)
(72) Erfinder: IZBICKI, Jakob, c/o Universität Hamburg, 20246 Hamburg (DE); HOSCH, Stefan, c/o Universität Hamburg, 20246 Hamburg (DE); SCHEUNEMANN, Peter, c/o Universität Hamburg, 20246 Hamburg (DE)
(74) Vertreter: Meissner, Bolte & Partner
(86) Internationale Anmeldenummer: PCT/DE2002/000532
(87) Internationale Veröffentlichungsnummer: WO 2002/064754

(56) Entgegenhaltungen:
- HOSCH STEFAN ET AL: "Malignant potential and cytogenetic characteristics of occult disseminated tumor cells in esophageal cancer." CANCER RESEARCH, Bd. 60, Nr. 24, 15. Dezember 2000 (2000-12-15), Seiten 6836-6840, XP002215092 ISSN: 0008-5472
- PANTEL KLAUS ET AL: "Establishment of Micrometastatic Carcinoma Cell Lines: A Novel Source of Tumor Cell Vaccines." JOURNAL OF THE NATIONAL CANCER INSTITUTE (BETHESDA), Bd. 87, Nr. 15, 1995, Seiten 1162-1168, XP000611729 ISSN: 0027-8874
- HOMBAUER H ET AL: "Selective interactions between epithelial tumour cells and bone marrow mesenchymal stem cells." BRITISH JOURNAL OF CANCER, Bd. 82, Nr. 7, April 2000 (2000-04), Seiten 1290-1296, XP000179469 ISSN: 0007-0920
- DATABASE INTERNET [Online] www.pvi.uni-bonn.de/medien.htm, "Zellkuturmedium und Herstellung eines Komplettmediums" XP002201652
- KUREC A S ET AL: "USE OF THE APAAP METHOD IN THE CLASSIFICATION AND DIAGNOSIS OF HEMATOLOGIC DISORDERS" CLINICS IN LABORATORY MEDICINE, Bd. 8, Nr. 1, 1988, Seiten 223-236, XP001079485 ISSN: 0272-2712

## Beschreibung

Die vorliegende Erfindung betrifft ein Medium zur Kultivierung von Tumorzellen, das die folgenden Bestandteile umfaßt: (a) fötales Kälberserum (FCS), (b) Penicillin-Streptomycin, (c) L-Glutamin, (d) Transferrin, (e) Insulin, (f) humaner epidermaler Wachstumsfaktor (EGF) und (g) α-transforming growth factor (α-TGF). Daneben betrifft die vorliegende Erfindung ein Verfahren zur Charakterisierung von Tumorzellen bzw. potentiellen Tumorzellen, das die Züchtung der Zellen in dem erfindungsgemäßen Medium umfaßt.

Durch den Einsatz von immunhistochemischen und molekularbiologischen Untersuchungstechniken ist der Nachweis epithelialer Tumorzellen in Lymphknoten möglich geworden (Byrne et al., J.Surg.Oncol. 13 (1987), 409-411; Calaluce et al., J.Surg.Oncol. 67 (1998), 194-202; Latza et al., J.Clin.Pathol. 43 (1990), 213-219; Liefers et al., NEJM 339 (1998), 223-228; Pantel et al., Prog.Histochem.Cytochem. 30 (1996), 1-60; Passlick et al., J.Clin.Oncol. 12 (1994), 1827-1832; Raymond et al., Pathology 21 (1989), 11-50; und Sidransky, Science 278 (1997), 1054-1059). Trotz einer immer größer werdenden Anzahl von Publikationen, die eine prognostische Relevanz von nodal frühdisseminierten Tumorzellen bei Patienten mit Karzinomen der Lunge, Brust, Prostata und des Gastrointestinaltraktes nachweisen konnten (lzbicki et al., NEJM 337 (1997), 1188-1194; Hosch et al., Pancreas 15 (1997), 154-159; Passlick et al., J.Clin.Oncol. 12 (1994), 1827-1832; Greenson et al., Cancer 73 (1994), 563-569; und Liefers et al., NEJM 339 (1998), 223-228), bleibt die biologische Relevanz von immunhistochemisch nachweisbaren, individuellen Tumorzellen aber weiterhin unklar. Dies beruht darauf, daß eine weiterführende Charakterisierung dieser Zellen aufgrund ihrer extrem niedrigen Häufigkeit bisher kaum möglich ist (1 Tumorzelle vor dem Hintergrund von 10⁴ - 10⁵ normalen Lymphknotenzellen). Die mit den bisherigen Untersuchungsverfahren einhergehende Unsicherheit, ob es sich bei individuellen Tumorzellen um tumorerzeugende Mikrometastasen oder aber lediglich um abgeschilferte und avitale Zellen des Primärtumors handelt, verhindert derzeit die Aufnahme dieses Parameters in die internationale "Tumorstaging"-Nomenklatur, d.h. dies läßt auch keine Prognosen bzw. die evtl. nötige Ergreifung weiterer therapeutischer Maßnahmen zu.

Hosch et al. (Cancer Research, Bd. 60, Nr. 24, S. 6836-6840 (2000)) beschreiben die Kultivierung von Krebszellen der Speiseröhre (Ösophagus-Adenokarzinom).

Pantel et al. (Journal of the National Cancer Institute, Bd. 87, Nr. 15, S. 1162-1168 (1995)) beschreiben ein Medium zur Kultivierung von Tumorzellen, dessen Basismedium das weit verbreitete RPMI 1640-Medium ist.

Hombauer et al. (British Journal of cancer, Bd. 82, Nr. 7, S. 1290-1296 (2000)) zeigen ein Medium zur Kultivierung von Tumorzellen, dessen Basismedium alpha-MEM (modified essential medium) ist. Supplementiert wird das Basismedium mit 20% fötalem Kälberserum.

Somit liegt der vorliegenden Erfindung das technische Problem zugrunde, diese Nachteile der im Stand der Technik angewandten Verfahren zu überkommen, d.h. Mittel bereitzustellen, die einen Nachweis darüber erlauben, ob es sich bei potentiellen Tumorzellen in einer Gewebeprobe um lebensfähige und maligne Tumorzellen handelt.

Die Lösung dieses technischen Problems erfolgt durch die Bereitstellung der in den Patentansprüchen gekennzeichneten Ausführungsformen.

Es wurde überraschenderweise gefunden, daß Zellen mit unklarem Status hinsichtlich Vitalität und Tumorerzeugung in dem erfindungsgemäßen Medium vermehrt und hinsichtlich möglicher Tumorerzeugung weiter untersucht werden können. Dazu wurden Gewebeproben aus einem konventionell histopathologisch tumorfreien, immunhistochemisch aber positiven Lymphknoten eines Patienten mit Ösophagus-Adenokarzinom in dem erfindungsgemäßen Medium kultiviert, um so die Biologie von frühdisseminierten Tumorzellen in Lymphknoten besser untersuchen zu können. Es konnte eine permanente Zellinie (LNHOS1) etabliert werden. Durch Züchtung in dem erfindungsgemäßen Medium gelang auch die Etablierung einer permanenten Zellinie aus dem autologen Primärtumor des Patienten (PTHOS1). Beide Zellinien wurden unter **Verwendung von Mikrosatelliten-Analyse und** HLA-DRBI*-Typisierung, Multiplex-Fluoreszenz-in situ-Hybridisierung (M-FISH), Fluoreszenz-aktivierter Durchflußzytometrie (FACS) und ELISA-Technik vergleichend näher charakterisiert. Diese Analysen ergaben für die aus dem Lymphknoten generierte Zellinie LNHOS1 tumorspezifische zytogenetische Alterationen sowie die Expression von metastasierungs-assoziierten Proteinen. Darüber hinaus zeigte sich nach Xenotransplantation in immundefiziente SCID-Mäuse, daß diese Zellinie tumorerzeugend und metastatisch ist. Mit der erfindungsgemäßen Zellinie LNHOS1 ist somit erstmalig der direkte Nachweis erbracht worden, daß es sich bei immunhistochemisch nachweisbaren individuellen Tumorzellen (okkult disseminierte Tumorzellen) durchaus um vitale und maligne Tumorzellen handelt. Zum einen beweist der mittels Züchtung in dem erfindungsgemäßen Medium ermöglichte Nachweis einer permanenten Expansion von "okkulten Tumorzellen", daß es sich bei derartigen Zellen um Zellen mit einem unbegrenzten proliferativen Potential in vitro handeln kann, zum anderen zeigen diese Zellen in vivo im SCID-Maus-Modell tumorerzeugendes und metastatisches Potential. Durch weiterführende Analysen solcher Zellinien können so wichtige Informationen über die biologischen Eigenschaften von frühdisseminierten Tumorzellen gewonnen werden, die hinsichtlich einer gegebenenfalls erforderlichen Therapie einer minimalen residualen Tumorerkrankung bzw. auch darüber hinaus bei der Entwicklung neuer Strategien zum Nachweis und zur Therapie einer solchen Erkrankung hilfreich sind.

Insbesondere betrifft die vorliegende Erfindung ein Medium zur Kultivierung von Tumorzellen, das dadurch gekennzeichnet ist, daß es die folgenden Bestandteile umfaßt: (a) fötales Kälberserum (FCS), (b) Penicillin-Streptomycin, (c) L-Glutamin, (d) Transferrin, (e) Insulin, (f) humaner epidermaler Wachstumsfaktor (EGF) und (g) α-transforming growth factor (α-TGF). Die geeigneten Konzentrationen der einzelnen Bestandteile können vom Fachmann leicht ermittelt werden, wobei die einzelnen Bestandteile des erfindungsgemäßen Mediums in folgenden Konzentrationsbereichen vorliegens: (a) 2 - 20% fötales Kälberserum (FCS), (b) 20 - 2000 E/ml Penicillin-Streptomycin, (c) 0,2 - 20 mM L-Glutamin, (d) 2 - 200 µg/ml Transferrin, (e) 0,5 - 50 µg/ml Insulin, (f) 2 - 200 ng/ml humaner epidermaler Wachstumsfaktor (EGF) und (g) 2 - 200 ng/ml α-TGF.

Das Medium kann ggf. noch weitere Bestandteile, wie z.B. "basic fibroblast growth factor", Zytokine (z.B. Interleukine, Interferon) oder andere Wachstumsfaktoren.

Besonders bevorzugt ist ein erfindungsgemäßes Medium mit folgenden Konzentrationen der einzelnen Bestandteile: (a) 10% fötales Kälberserum (FCS), (b) 200 IU/ml Penicillin-Streptomycin, (c) 2 mM L-Glutamin, (d) 20 µg/ml (humanes) Transferrin, (e) 5 µg/ml (bovines) Insulin, (f) 50 ng/ml (rekombinanter) humaner epidermaler Wachstumsfaktor (EGF) und (g) 20 ng/ml α-TGF.

Das "Basismedium", dem die erfindungsgemäßen Bestandteile zugegeben werden, kann jedes Medium sein, das üblicherweise für die Züchtung von Tierzellen, vorzugsweise Säugerzellen verwendet wird, beispielsweise RPMI 1640 + 5-20% FCS (s. DSMZ-Katalog, Human and Animal Cell Lines, 1999) oder TUM-Medium.

In einer bevorzugten Ausführungsform des erfindungsgemäßen Mediums ist das "Basismedium", in dem die vorstehenden Bestandteile (a) bis (g) enthalten sind, RPMI 1640-Medium (Life Technologies, Paisley, Schottland).

Das erfindungsgemäße Medium hat den Vorteil, daß nach Disaggregation von z.B. Lymphknoten-Proben regelmäßig eine Monolayer-Bildung von zunächst fibroblastoiden Zellen beobachtet werden konnte. Nach 3 bis 6 Wochen Bebrütunbg ließen sich dann Tumorzellnester nachweisen. Ebenfalls konnte beobachtet werden, daß sich diese Tumorzellen in enger räumlicher Beziehung zu den fibroblastoiden Zellen befanden, was impliziert, daß sich diese kokultivierten Zellen günstig auf das Wachstum der Tumorzellen auswirken, indem sie als "by-stander"-Zellen fungieren können.

Die vorliegende Erfindung betrifft außerdem ein Verfahren zur Charakterisierung von Tumorzellen bzw. vermeintlichen Tumorzellen, das dadurch gekennzeichnet ist, daß man die Zellen in dem erfindungsgemäßen Medium unter geeigneten Bedingungen, gegebenenfalls unter (teilweiser) Erneuerung des Mediums in geeigneten Zeitabständen, züchtet, die Zellen erntet und dann charakterisiert. Geeignete Bedingungen, beispielsweise hinsichtlich geeigneten Behältern, Temperatur, relativer Luftfeuchte, O₂-Gehalt und CO₂-Gehalt der Gasphase sind dem Fachmann bekannt. Vorzugsweise werden die Tumorzellen bzw. die vermeintlichen Tumorzellen in dem erfindungsgemäßen Medium unter den folgenden Bedingungen kultiviert: (a) 37°C, (b) 100% rel. Luftfeuchte, (c) 10% O₂ und (d) 5% CO₂. Geeignete Verfahren zur (genotypischen und phänotypischen) Charakterisierung der Zellen, die eine Aussage hinsichtlich ihres möglichen tumorerzeugenden und/oder metastasenbildenden Potentials erlauben, sind dem Fachmann bekannt. Dazu zählen beispielsweise die in dem nachstehenden Beispiel 2 beschriebenen in vitro-und in vivo-Verfahren.

Im Rahmen der Erfindung konnten die beiden Zellinien LNHOS1 und PTHOS1 durch Züchtung in dem erfindungsgemäßen Medium etabliert werden.

Gemäß Budapester Vertrag wurden die folgenden Zellkulturen bei der Deutschen Sammlung von Mikroorganismen und Zellkulturen GmbH (DSMZ), Mascheroder Weg 1b, 38124 Braunschweig, Deutschland hinterlegt:

| | | |
|---|---|---|
| Zellkultur LN HOS1 | DSM ACC2472 | 2. November 2000 |
| Zellkultur PT HOS1 | DSM ACC2471 | 14. Sept. 2000 |

Die nachstehenden Beispiele erläutern die Erfindung.

### Beispiel 1

### Etablierung der Zellinien PTHOS1 und LNHOS1

Sofort nach Entnahme des Operationspräparates, das von einem Patienten mit Ösophagus-Krebs (Stadium pT₁pN₁M₀ entsprechend der internationalen "Tumorstaging"-Nomenklatur) stammte, wurden Gewebeproben aus dem Primärtumor und fünf verschiedenen Lymphknoten-Stationen entnommen, welche entsprechend eines Kartierungsschemas benannt wurden. Anschließend wurde jeder Lymphknoten wie in Izbicki et al., NEJM 337 (1997), 1188-1194, beschrieben halbiert. Eine Hälfte des Lymphknotens gelangte zur routinemäßigen histopathologischen Begutachtung, die andere Hälfte und die Primärtumorprobe wurden für die immunhistochemischen Analysen in flüssigem Stickstoff schockgefroren und bis zur weiteren Verarbeitung bei -80°C aufbewahrt. Zusätzlich wurden aus einem der makroskopisch unauffälligen Lymphknoten an der Arteria gastrica sinistra Proben für die Zellkultur entnommen. Diese Gewebeproben wurden in kleine Stücke geschnitten, wiederholt in RPMI 1640-Medium (Life Technologies, Paisley, Schottland) gewaschen und mit Hilfe einer "medimashine" (Fa. DAKO, Hamburg) zu Einzelzellsuspensionen disaggregiert. Die Zellsuspensionen wurden in Extrazellularmatrix-(ECM-) beschichteten T25 Zellkulturflaschen bei 37°C und 5%C0₂ im Brutschrank (Inkubator B 5061, Heraeus, München, Deutschland) inkubiert. Die Expansion dieser Zellinien erfolgte in dem mit den folgenden Bestandteilen supplementierten RPMI 1640-Medium: 10% fötales Kälberserum (FCS; c.c.pro, Neustadt, Deutschland)), 200 E/ml Penicillin-Streptomycin (Ausgangskonzentration: 10000 E/ml; Life Technologies, Karlsruhe, Deutschland), 2 mM L-Glutamin (Ausgangskonzentration: 200 mM; Life Technologies, Karlsruhe, Deutschland), 20 *µ*g/ml Transferrin (Boehringer, Mannheim, Deutschland), 5 *µ*g/ml Insulin (Boehringer, Mannheim, Deutschland), 20 ng/ml humaner epidermaler Wachstumsfaktor (EGF) (PBH, Hannover, Deutschland) und 20 ng/ml α-TGF. Die für die Zugabe zum Medium erforderliche Verdünnung von α-TGF erfolgte in PBS, das mit 1% Rinderserumalbumin (BSA) supplementiert worden war. Diese beiden Zellinien ließen sich bisher über 70 Passagen permanent expandieren und können daher als immortal gelten.

### Beispiel 2

### Charakterisierung von PTHOS1 und LNHOS1 als tumorerzeugende und metastatische Zellinien

In der für immunhistochemische Analysen kryokonservierten Lymphknoten-Probe konnten mittels APAAP-Technik 3 individuelle Tumorzellen vor dem Hintergrund von ca. 10⁵ normalen Lymphknotenzellen nachgewiesen werden. Hierfür wurden Kryoschnitte nach einstündiger Luftrocknung für eine Minute in +4°C kaltem Aceton fixiert und anschließend in Tris-gepufferter physiologischer Kochsalzlösung (TBS, pH 7,4) rehydriert. Anschließend wurden die Präparate für 20 Minuten mit AB-Serum (Biotest Diagnostics 805135, Dreieich, Deutschland) 1:10 in TBS verdünnt, inkubiert, um eine unspezifische Hintergrundfärbung zu verhindern. Danach erfolgte die Inkubation mit dem monoklonalen anti-Epithelzell-Antikörper Ber-EP4 (Code-Nr. M0804, Dako, Hamburg; Latza et al., J.Clin.Pathol. 43 (1990), 213-219) in einer Verdünnung von 1:400 in TBS (0,625 µg/ml). Nach dreimaligem Waschen in TBS für jeweils 5 Minuten wurde ein Kaninchen-anti-Maus-Ig-Brückenantikörper (Code-Nr. Z0259, Dako, Hamburg), 1:50 in TBS verdünnt (64 µg/ml) für 30 Minuten inkubiert. Nach erneutem Waschen für 3 mal 5 Minuten in TBS wurde der APAAP-Komplex (Code-Nr. D0651, Dako, Hmaburg) in einer Verdünnung von 1:100 in TBS (0,9 µg/ml) für 30 Minuten appliziert. Die Darstellung der Antikörper-Reaktion erfolgte mittels Neufuchsin (s. APAAP-Substratansatz). Nach dreißigminütiger Inkubation der Schnittpräparate im Substrat folgten das einmalige Waschen in Aqua dest und die Kerngegenfärbung mit Gill's Hämatoxylin für 30 Sekunden (Passlick et al., Ann. Thorac. Surg. 61, S. 77-83, (1996)).

Der autologe Ursprung beider Zellinien wurde durch eine Mikrosatelliten-Analyse bestätigt, für die der "GenePrint Fluorescent CTTv STR^{™} Multiplex-Systems"-Kit von Promega (Madison, W1, USA) verwendet wurde. Alle DNA-Amplifikate wiesen bei der elektrophoretischen Auftrennung auf Polyacrylamidgelen ein identisches Mikrosatelliten-Bandenmuster auf. Um eine Kontamination während Passagierung und Mediumwechsel durch artfremde Zellen auszuschließen, wurden mehrfach HLA-DRBI*-Typisierungen nach dem "oligonucleotid typing"-System von Nevinny-Stickel et al. (Hum. Immunol. 31: S. 7-13 (1991)) durchgeführt. In allen Fällen zeigten die Zellinien und die Sublinien einen identischen Genotyp.

Beide Zellinien wurden außerdem unter Verwendung der M-FISH-Technik umfangreich zytogenetisch charakterisiert. Diese Technik ermöglicht die simultane Darstellung aller menschlichen Chromosomen in verschiedenen Farben (Speicher et al., Nature Genetics, 12 (1996), S. 368-375; Eils et al., Cytogenet. Cell Genet., 82 (3-4), S. 160-171 (1998)). Für die Analyse werden Metaphasen-Chromosomen der Zellinien präpariert. Hierbei wird nach Standardprotokollen verfahren. Die gespreiteten Metaphasen werden mit RNase und Pepsin vorbehandelt (Lengauer et al., Cancer Res. 52, S. 2590-2596 (1992)). Bei der nachfolgenden M-FISH-Analyse wird wie in Speicher et al. (1996, s.o.) und Eils et al. (1998, s.o.) beschrieben vorgegangen. Durchflußzytometrisch gewonnene "whole chromosome painting"-Sonden (J. Wienberger, Cambridge) werden nach einem Standard-DOP-PCR-Protokoll amplifiziert (Telenius et al., Genes Chromosomes Cancer, (1992), 4, S. 257-263). Um alle 24 menschlichen Chromosomen in verschiedenen Farben darstellen zu können, werden 5 verschiedene Sonden-Pools hergestellt (jeder für sich gegen einen unterschiedlichen Satz an Chromosomen gerichtet) und unter Verwendung eines DOP-PCR-Assays mit verschiedenen Fluorochromen markiert (Eils et al., 1998, s. o.). Bei der Ethanol-Präzipitation dieser Sonden wird Cot-1 DNA und Lachs-Sperma-DNA zugesetzt. Die Hybridisierung des Sonden-Mixes mit der zu analysierenden Target-DNA dauert 2 Tage. Bei der Visualisierung der Hybridisierungs-Ergebnisse wurde nach einenm Standard-Protokoll verfahren (Speicher et al., 1998, s.o.; Eils et al., 1998, s.o.). Auswertung und Dokumentation kann mit einem Leica DMRXA-RF8 Mikroskop, welches mit einer Sensys-Kamera (Photometrics, Tuscon, USA) und einer Leica Software ausgerüstet ist (Leica Microsystems Imaging Solutions Ltd., Cambridge, Großbritannien), erfolgen. Die Ergebnisse können sowohl in digitalen Echt- als auch in Falschfarbenbildern zur Darstellung gebracht werden (Eils et al., 1998, s.o.).

Als Ergebnis wurde erhalten, daß beide Zellinien über einen hypertriploiden Chromosomensatz verfügen. Einige strukturelle Veränderungen, wie del (5) (q1?3q2?22), der(8), der(9)del(9)(p21-22)del(9)(q21-22), der(17), der(22)t(19;22) und der Verlust des Y-Chromosoms wurde in allen oder den meisten der untersuchten Metaphase-Präparate beider Zellinien nachgewiesen. Weitere Abberationen wie der(1)t(1;20), der(3)t(3;17), der(8)t(5;8), der (10) t (1;10) und der(13)t(5;13) wurden ausschließlich in etwa 50% aller Metaphase-Spreitungen von PTHOS1-Zellen beobachtet, während LNHOS1-Zellen diese chromosomalen Anomalien nicht aufwiesen. Andererseits war eine weitere strukturelle Veränderung, und zwar eine Insertion von Chromosom-13-Material in den kurzen Arm des Chromosom 1 [ins (1;13) (p22;q?)], ausschließlich bei LNHOS1-Zellen nachzuweisen. PTHOS1-Zellen zeigten diese Veränderung dagegen nicht. Nach der DAPI-Bänderung zu urteilen, stammt dieses inserierte Chromosom 13-Material vermutlich von einer hellen G-Bande.

Desweiteren erfolgte eine umfangreiche phänotypische Charakterisierung beider Zellinien durch indirekte Immunfluoreszenz-Technik unter Verwendung eines "FACScan cell sorters" (Becton Dickinson., San Jose, CA, USA) (siehe Tabelle 1). Um den epithelialen Ursprung beider etablierter Zellinien zu bestätigen, wurde die Expression von Histogenesemarkern wie Zytokeratinen, Vimentin, α-smooth-muscle actin und endothelialen Antigenen (Faktor VIII, EN-4) mittels Durchflußzytometrie analysiert. Im Einklang mit der Entdifferenzierung des in situ-Primärtumors ließ sich eine Expression von epithelialen Zytoskelett-Proteinen (Zytokeratine) für PTHOS1 mit den monoklonalen Anti-Zytokeratin-Antikörpern (mAk) A45B/B3 (Fa. Micromet, München), AE1/AE3 (Fa. DAKO, Hamburg), CK2 (Fa. Roche Diagnostics. Mannheim) und LP34 5D3 (Fa. Medac, Hamburg) nicht nachweisen, während LNHOS1 ein heterogenes Expressionsprofil aufwies. Weder PTHOS1- noch LNHOS1-Zellen reagierten mit den mAk gegen Endothelialzellen oder α-smooth-muscle actin, während das mesenchymale Zytoskelett-Protein Vimentin heterogen von beiden Zellinien exprimiert wurde. Dies legt eine "epithelial to mesenchymal transition" (EMT) nahe, welche mit einer Erhöhung der Motilität von Karzinomzellen einhergeht (Gilles et al, Breast J. 2 (1996), 83-96). Für den epithelialen Ursprung beider Zellinien spricht weiterhin die Expression von Proteinen des E-Cadherin-Catenin-Komplexes, welcher hauptverantwortlich für die homotypische Zell-Zell-Adhäsion epithelialer Zellen ist. Andererseits wird das epitheliale Zelladhäsionsmolekül EpCAM (17-1A) nur von einer LNHOS1-Subpopulation exprimiert, während PTHOS1-Zellen dieses Molekül nicht exprimierten. Beide Zellinien weisen charakteristische Merkmale maligner Tumorzellen auf, wie die Überexpression des erbB2-Onkogenproduktes p185^{erbB2} und des p53-Tumorsuppressorgen-Produktes. Überexpression von erbB2 und Akkumulation des p53-Proteins sind bei epithelialen Tumoren, einschließlich dem Ösophaguskarzinom, häufige Veränderungen. Da für eine p53-Überexpression häufig Mutationen im p53-Gen verantwortlich sind, wurden außerdem die p53-Gene beider Zellinien sequenziert. Dabei wurde eine Punktmutation im Codon 176 [CGC(Arg)→CAC(His)] - einer "hot spot"-Region - nachgewiesen, welche die Expression eines stabilisierten p53-Proteins bewirkt (Harris, JNCI 88 (1996), 1442-1455).

Desweiteren wurde bei beiden Zellinien die Expression von Molekülen untersucht, die bei einigen Tumorentitäten in den Prozeß von Progression und Metastasierung involviert sind. In diesem Zusammenhang spielen Adhäsionsmoleküle durch Vermittlung von hetero- und homotypischen Zell-Zell- bzw. Zell-Matrix-Interaktionen eine entscheidende Rolle. In Bezug auf die Zell-Matrix-Adhäsionsmoleküle ließ sich auf beiden Zellinien eine Expression von α₅- (CD49e), α₆- (CD49f) und β₁-Integrin-Untereinheiten nachweisen, während die Integrin-Untereinheiten αᵥ (CD51), ß₂ (CD18) und ß₃ (CD61) weder von LNHOS1- noch von PTHOS1-Zellen exprimiert wurden. Dagegen wurde die ß₄-Integrin-Untereinheit (CD104) interessanterweise ausschließlich von einer LNHOS1-Subfraktion exprimiert. Ein weiteres Zell-Matrix-Adhäsionsmolekül, der 67kD Laminin-Rezeptor (67LR), wurde ebenfalls ausschließlich von einer LNHOS1-Subpopulation exprimiert. Dieser Rezeptor spielt eine wichtige Rolle bei der Interaktion zwischen Tumorzellen und der Basalmembran. Obwohl E-Cadherin und seine intrazytoplasmatischen Liganden α- und γ-Catenin (Plakoglobin) von beiden Zellinien exprimiert wurden, wurde Desmoglein (DMG) als wichtigste Komponente des desmosomalen Membrankerns wiederum nur von einer Subpopulation von LNHOS1-Zellen exprimiert, während PTHOS1-Zellen keine Expression von Desmoglein aufwiesen. Auch für die Adhäsionsmoleküle der Immunglobulin-Superfamilie war eine differente Expression zwischen PTHOS1 und LNHOS1-Zellen nachzuweisen. Während beide Zellinien in der Durchflußzytometrie-Analyse positiv für LFA-3 (CD58) bzw. negativ für KAI-1 (CD82), VCAM-1 (CD106) und Muc-18 (CD146) waren, konnte eine Neoexpression des normalerweise nur von lymphoiden und myeloiden Zellen exprimierten ICAM-1 (CD54) wiederum nur auf einer 25% umfassenden LNHOS1-Subpopulation nachgewiesen werden.

Zusätzlich zu Zell-Zell- und Zell-Matrix-Interaktionen, die über Adhäsionsmoleküle vermittelt werden, benötigen Tumorzellen zur Durchwanderung des umliegenden Stromas die Fähigkeit zum proteolytischen Abbau der Extrazellularmatrix (ECM). In diesem Zusammenhang spielen Metallomatrixproteasen (MMPs) und ihre Inhibitoren eine bedeutende Rolle. MMPs ermöglichen die Zell-Migration durch Abbau und Regulation spezifischer ECM-Komponenten, wie Laminin, Kollagen, Fibronektin oder Vitronektin, und werden ihrerseits durch Bindung von "tissue inhibitors of metalloproteases" (TIMPs) inhibiert. Beide Zellinien exprimierten MMP-9 (Gelatinase B), MMP-14 (MT-MMP) sowie TIMP-1, während eine Expression von MMP-2 (Gelatinase A), MMP-3 (Stromelysin) oder TIMP-2 weder für PTHOS1 noch für LNHOS1 nachweisbar war. Bei der Analyse des Blutgruppen-Antigens Lewis Y (Le^{y}), welches auf Tumoren häufig überexprimiert wird, war eine Expression ebenfalls nur bei einer Subpopulation von LNHOS1-Zellen nachweisbar.

Um das tumorerzeugende Potential von PTHOS1- und LNHOS1-Zellen in vivo zu prüfen, wurden zwischen 1x10⁵ und 6,5x10⁶ Tumorzellen subcutan (s.c.) in die Flanken von immundefizienten SCID-Mäusen injiziert. Nach einer Beobachtungszeit von 2 bis 29 Wochen entwickelten alle 20 Mäuse, denen PTHOS1-Zellen appliziert worden waren und 7 von 8 Mäusen, denen LNHOS1-Zellen appliziert worden waren, lokale Tumoren im Bereich der Applikationsstelle. Dabei waren lokale Tumorformationen nach Injektion von PTHOS1-Zellen deutlich früher sichtbar, als nach Injektion von LNHOS1-Zellen (im Durchschnitt 4,7 vs. 13,9 Wochen, p=0,0001). Ebenso benötigten PTHOS1 Zellen für die Entwicklung maximal großer lokaler Tumoren (>15mm im Durchmesser) deutlich weniger Zeit als LNHOS1 Zellen (im Durchschnitt 8,6 vs. 18,7 Wochen, p=0.002). Bei je zwei Tieren aus beiden Gruppen kam es zu einer lokalen Tumorpenetration durch das Peritoneum in die Bauchhöhle, während eine makroskopische Metastasierung autoptisch in keinem Fall nachgewiesen werden konnte. Andererseits gelang der immunhistochemische Nachweis einer pulmonalen Mikrometastasierung sowie die Re-Generierung mehrerer Zellinien aus verschiedenen Mausorganen (mesenteriale Lymphknoten, Knochenmark, Lunge).

**Tabelle 1: Phänotypische Charakteristika der Zellinien LNHOS1 und PTHOS1**

| **Antigen-Familie** | | **positiv** | **negativ** | |
|---|---|---|---|---|
| Epitheliale | | E-Cadherin | | |
| Adhäsions- | | (Desmoglein*) | | |
| moleküle | | α-Catenin | | |
| | | Plakoglobin | | |
| | | (EpCAM*) | | |
| | | Muc-1 | | |
| | | (67 kD Lamininrezeptor*) | | |
| Integrine | | α₅ | | αᵥ |
| | | α₆ | | β₂ |
| | | β₁ | | β₃ |
| | | (β₄*) | | |
| Ig-Superfamilie | (ICAM-1*) | | KAI-1 | |
| | | LFA-3 | | VCAM-1 |
| | | | | Muc-18 |
| Metallomatrix- | | MMP-9 | | MMP-2 |
| Proteinasen und | MMP-14 | | MMP-3 | |
| Inhibitoren | | TIMP-1 | TIMP-2 | |
| Proto-Onkogen-/ | erbB2 | | EGF-R | |
| Suppressorgen- | (p53) | | | |
| Produkte | | | | |
| Histogenese- | | (Zytokeratine*) | α-smooth muscle | |
| Marker | | | | actin |
| | | (Vimentin) | | Faktor VIII |
| | | | | EN-4 |
| andere Moleküle | β₂-Mikroglobulin | | | |
| | | (Lewis Y*) | | |

| | | | | |
|---|---|---|---|---|
| ( ), heterogene Population mit positiven und negativen Zellen *, ausschließlich von LNHOS1-Zellen exprimierte Moleküle | | | | |

## Patentansprüche

1. Medium zur Kultivierung von Tumorzellen, **dadurch gekennzeichnet, daß** es die folgenden Bestandteile umfaßt:
(a) fötales Kälberserum (FCS);
(b) Penicillin-Streptomycin;
(c) L-Glutamin;
(d) Transferrin;
(e) Insulin;
(f) humaner epidermaler Wachstumsfaktor (EGF); und
(g) α-TGF,
wobei die Bestandteile (a) bis (g) in folgenden Konzentrationen vorliegen:
(a) 2 - 20%;
(b) 20 - 2000 E/ml;
(c) 0,2 - 20 mM;
(d) 2 - 200 µg/ml;
(e) 0,5 - 50 µg/ml;
(f) 2 - 200 ng/ml; und
(g) 2 - 200 ng/ml.

2. Medium nach Anspruch 1, wobei die Bestandteile (a) bis (g) in folgenden Konzentrationen vorliegen:
(a) 10%;
(b) 200 E/ml;
(c) 2 mM;
(d) 20 *µ*g/ml;
(e) 5 *µ*g/ml;
(f) 50 ng/ml ; und
(g) 20 ng/ml.

3. Medium nach Anspruch 1 oder 2, wobei das Basismedium für die Zugabe der Bestandteile (a) bis (g) RPMI 1640-Medium ist.

4. Verfahren zur Charakterisierung von Tumorzellen oder potentiellen Tumorzellen, **dadurch gekennzeichnet, daß** man die Zellen in dem Medium nach einem der Ansprüche 1 bis 3 unter geeigneten Bedingungen kultiviert, die Zellen gewinnt und im Anschluß daran charakterisiert.

5. Verfahren nach Anspruch 4, weiter **dadurch gekennzeichnet, daß** die Tumorzellen unter folgenden Bedingungen kultiviert werden:
(a) 37°C;
(b) 100% rel. Luftfeuchte;
(c) 10% O₂; und
(d) 5% CO₂.

## Claims

1. Medium for cultivating tumor cells, **characterized in that** it comprises the following constituents:
(a) fetal calf serum (FCS);
(b) penicillin-streptomycin;
(c) L-glutamine;
(d) transferrin;
(e) insulin;
(f) human epidermal growth factor (EGF); and
(g) α-TGF,
wherein the constituents (a) through (g) are present in the following concentrations:
(a) 2 - 20%;
(b) 20 - 2000 U/ml;
(c) 0.2 - 20 mM;
(d) 2 - 200 µg/ml;
(e) 0.5 - 50 µg/ml;
(f) 2 - 200 ng/ml; and
(g) 2 - 200 ng/ml.

2. Medium according to claim 1, wherein the constituents (a) through (g) are present in the following concentrations:
(a) 10%;
(b) 200 U/ml;
(c) 2 mM;
(d) 20 µg/ml;
(e) 5 µg/ml;
(f) 50 ng/ml; and
(g) 20 ng/ml.

3. Medium according to claim 1 or claim 2, wherein the base medium for the addition of constituents (a) through (g) is the RPMI-1640 medium.

4. Method of characterizing tumor cells or potential tumor cells, **characterized in that** the cells are cultivated in the medium according to any one of claims 1 through 3 under appropriate conditions, the cells are recovered and subsequently **characterized**.

5. Method according to claim 4, further **characterized in that** the tumor cells are cultivated under the following conditions:
(a) 37°C;
(b) 100% relative air humidity;
(c) 10% O₂; and
(d) 5% CO₂.

## Revendications

1. Milieu destiné à cultiver des cellules tumorales, **caractérisé en ce qu'**il comprend les éléments constitutifs suivants :
(a) sérum de veau foetal (FCS) ;
(b) pénicilline - streptomycine ;
(c) glutamine L ;
(d) transferrine ;
(e) insuline ;
(f) facteur de croissance de l'épiderme humain (EGF) ; et
(g) TGF α,
dans lequel les éléments constitutifs (a) à (g) sont présents dans les concentrations suivantes :
(a) 2 à 20% ;
(b) 20 à 2000 U/ml ;
(c) 0,2 à 20 mM ;
(d) 2 à 200 µg/ml ;
(e) 0,5 à 50 µg/ml ;
(f) 2 à 200 ng/ml ; et
(g) 2 à 200 ng/ml.

2. Milieu selon la revendication 1, dans lequel les éléments constitutifs (a) à (g) sont présents dans les concentrations suivantes :
(a) 10 % ;
(b) 200 U/ml ;
(c) 2 mM ;
(d) 20 µg/ml ;
(e) 5 µg/ml ;
(f) 50 ng/ml ; et
(g) 20 ng/ml.

3. Milieu selon la revendication 1 ou 2, dans lequel le milieu de base pour l'adjonction des éléments constitutifs (a) à (g) est du fluide RPMI 1640.

4. Procédé de caractérisation de cellules tumorales ou de cellules tumorales potentielles, **caractérisé en ce qu'**on cultive les cellules dans le milieu selon l'une des revendications 1 à 3 dans des conditions appropriées, qu'on extrait les cellules et qu'on les caractérise suite à cela.

5. Procédé selon la revendication 4, **caractérisé en outre en ce que** les cellules tumorales sont cultivées dans les conditions suivantes :
(a) 37 °C ;
(b) 100 % d'humidité relative de l'air ;
(c) 10 % d'O₂ ; et
(d) 5 % de CO₂.
